# EUROPEAN PATENT SPECIFICATION

(11) **EP 2 195 419 B1**
(45) Date of publication and mention of the grant of the patent: **19.12.2012**
(21) Application number: 08835162.2
(22) Date of filing: 01.10.2008
(51) Int. Cl.: C12N 7/02

(54) **METHODS FOR DRYING BACTERIOPHAGE AND BACTERIOPHAGE-CONTAINING COMPOSITIONS**
TROCKNUNGSVERFAHREN FÜR BAKTERIOPHAGEN UND BAKTERIOPHAGENHALTIGE ZUSAMMENSETZUNGEN
PROCÉDÉS DE SÉCHAGE DE BACTÉRIOPHAGES ET COMPOSITIONS CONTENANT DES BACTÉRIOPHAGES

(30) Priority: 01.10.2007 US 976727 P
(43) Date of publication of application: 16.06.2010
(73) Proprietor: Omnilytics Incorporated, Salt Lake City, UT 84116 (US)
(72) Inventor: WALBECK, Alan, K., Salt Lake City, UT 84116 (US)
(74) Representative: Isarpatent
(86) International application number: PCT/US2008/078494
(87) International publication number: WO 2009/046138

(56) References cited:
- EP-A2- 0 403 292
- WO-A2-03/000274
- US-A- 5 149 653
- US-A- 5 149 653
- US-A- 5 792 625
- US-A1- 2003 234 012
- US-A1- 2005 050 759
- US-A1- 2005 050 759
- IIJIMA T ET AL: "A METHOD FOR PRESERVATION OF BACTERIA AND BACTERIO PHAGES BY DRYING", CRYOBIOLOGY, vol. 10, no. 5, 1973, pages 379-385, XP002594275, ISSN: 0011-2240
- COX C S ET AL: "VIABILITY AND ELECTRON MICROSCOPE STUDIES OF PHAGE T-3 AND PHAGE T-7 SUBJECTED TO FREEZE DRYING FREEZE THAWING AND AEROSOLIZATION", JOURNAL OF GENERAL MICROBIOLOGY, vol. 81, no. 1, 1974, pages 207-215, XP002594276, ISSN: 0022-1287
- WANG, LIANG ET AL.: 'Improvement of the Dissolution Rate of Nitrendipine Using a New Pulse Combustion Drying Method.' CHEM. PHARM. BULL. vol. 55, August 2007, page 1119, XP008131168

## Description

### TECHNICAL FIELD

The present invention relates generally to methods for drying bacteriophage and compositions that include bacteriophage. More specifically, the present invention relates to use of pulse combustion atomization spray drying processes to spray dry bacteriophage and bacteriophage-containing compositions.

### BACKGROUND OF RELATED ART

Bacteriophage may be produced using fermentation processes, in which liquid fermentation media, bacterial hosts, and bacteriophage are mixed with one another and incubated. Liquid lysate mixtures that include bacterial debris, fermentation media and an increased concentration bacteriophage result from such fermentation processes. In producing bacteriophage, the nature of the lysate and the interaction with the lysed host must be considered. Bacteriophage thrive within the fermentation environment, and typically continue to exist within that lysate for extended periods of time (with limited exceptions). If the bacteriophage is removed from the liquid lysate, or the environment is changed slightly, the viability of the bacteriophage could be compromised.

Additionally, liquids, such as water, promote the desired random interaction between bacteriophage and the targeted bacterial host. Accordingly, bacteriophage is typically applied in liquid form to substrates (e.g., plants, meats, etc.) that may be contaminated or infected with specific target bacteria.

As such, typically, liquid lysate solutions that result from bacteriophage fermentation processes are merely clarified to produce a final bacteriophage product, which is then shipped without further modification.

There are, however, situations where it would be more desirable to supply a dry bacteriophage product or a more compact (*e.g*., concentrated, etc.) bacteriophage product. An example of such a situation is when a host bacteria excretes a particular enzyme, protease, toxin, or other by-product that may harm the bacteriophage over time; *e.g*., by breaking down protein (which are the building blocks of bacteriophage), leading to the eventual demise of bacteriophage that remains within the liquid lysate solution (clarified or not). This breakdown occurs over time, even after the bacterial hosts have been killed by the bacteriophage, and can occur in as little as a few hours or as long as many days or even weeks. As a result, the production of liquid bacteriophage products has been limited to "well-behaved" bacterial hosts (those which do not excrete by-products that could harm the bacteriophage), which may limit the types or concentrations of certain types of bacteriophage that can be effectively produced on an industrial scale.

Various attempts have been made to produce dry bacteriophage products on a larger scale, including lyophilization (*i.e*., freeze-drying), spray drying, and vacuum drying processes (*see*, *e*.*g*., European patent application publication EP 0 403 292 (19 December 1990) of Microbial Developments Limited, page 4, lines 42-44 and 52-55). Nevertheless, the viability and virulence of the dried bacteriophage particles is unclear. Moreover, the susceptibility of bacteriophage particles to the conditions (*e.g*., temperatures, shear forces, etc.) of such processes have rendered some attempts largely unsuccessful, "killing" or otherwise diminishing the virulence of the vast majority of the bacteriophage particles. In fact, conventional spray dry processes typically result in products with at least a five log (10⁵) to eight log (10⁸) reduction in bacteriophage efficacy, representing a reduction in the effectiveness of the final product, compared to its pre-dried liquid form, of 99.999% to 99.999999%.

There are needs for bacteriophage and bacteriophage containing compositions that are in dry (*e.g*., powder, granules, etc.) form, for methods for drying bacteriophage and bacteriophage-containing compositions, and for methods for using dry bacteriophage and dry compositions that include bacteriophage.

### DISCLOSURE OF THE INVENTION

Bacteriophage are sensitive to shearing forces, extremely high temperatures (including the amount of time the bacteriophage is exposed to high temperatures) and other environmental variables. Due to the sensitive nature of bacteriophage, care may be taken to avoid damaging the anatomy of this microorganism. Of particular concern is keeping the portions of the bacteriophage requisite for adsorption, such as the tail fibers, intact. If these portions of the bacteriophage anatomy do not remain intact or viable, the bacteriophage will not be able to make contact and adsorb to the walls of the targeted bacteria strain.

The present invention, in one aspect, includes a method for manufacturing a dry bacteriophage preparation according to claim 1. In various embodiments, such processes may include subjecting a bulk liquid to pulse combustion drying. In addition to the bacteriophage, the bulk liquid may include at least one of a fermentation medium, a lysate and a host bacteria. In some embodiments, the bulk liquid may have a volume of up to twenty (20) liters.

A carrier material may also be introduced into the bulk liquid to increase its solids content. Examples of suitable carrier materials include dried milk, trehalose, and maltodextrin. In some embodiments, the solids content of the bulk liquid may be adjusted by at least 20%.

During pulse combustion drying, the bulk liquid may be subjected to a pressure of up to 0.1 bar. The contact temperature of the pulse combustion drying process may be 540° C. or less. An exit temperature during the pulse combustion drying process may be 82° C. or less. Pulse combustion drying may be effected at a rate of one liter every ninety (90) seconds or faster.

Pulse combustion drying processing of bacteriophage may maintain the efficacy and virulence of bacteriophage particles in a manner that reduces the number of viable bacteriophage particles, from processed liquid bacteriophage product to dry bacteriophage product, by no more than about one log (10¹).

Other aspects, as well as features and advantages, of the present invention will become apparent to those of skill in the art through consideration of the ensuing description and the appended claims.

### BEST MODE(S) FOR PRACTICING THE INVENTION

The present invention includes methods for forming dry bacteriophage products from liquid bacteriophage products, such as fermentation mixtures that include bacteriophage and, optionally, fermentation media, bacterial hosts for the bacteriophage, and/or bacterial debris, or "lysate." In such a method, a bulk liquid preparation including the bacteriophage (*i.e*., an industrial-scale quantity, which may be measured in gallons or liters) may be dried in fewer than ten hours and, in some embodiments, a few liters of liquid bacteriophage product (*e.g.*, up to about twenty liters) can be dried in as quickly as about thirty minutes, twenty minutes, or even ten minutes or less. Thus, drying may be effected at a rate of about one liter every minute and a half, about one liter per minute, or even two liters per minute.

When methods for forming dry bacteriophage products in accordance with teachings of the present invention are employed, the viability of a liquid bacteriophage product, or its virulence (*i.e*., the ability of bacteriophage to infect a host bacteria), is substantially maintained during the drying process. As an example, in some embodiments, the dry bacteriophage product that results from a drying process of the present invention may reduce the number of viable, or virulent, bacteriophage particles to no less than ten percent (10%) of the number of viable, or virulent, bacteriophage particles that were present in the initial, liquid bacteriophage product, representing no more than a one log (10¹) reduction in viable, or virulent, bacteriophage particles. In some embodiments, a two log (10²) reduction of viable, or virulent, bacteriophage particles may be acceptable, with the resulting dry bacteriophage product including at least one percent (1%) of the viable, or virulent, bacteriophage particles that were present in the initial, liquid bacteriophage product. In other embodiments, the reduction in viable phage particles may be as small as about 25% or less, with about 75% or more of the phage particles retaining their viability throughout the drying process.

As noted above, due to the delicate nature of bacteriophage (*e.g*., their tail fibers or other areas that are needed for the bacteriophage to effectively adsorb to a targeted host microorganism), the conditions (*e.g*., shear forces, temperatures, etc.) of conventional drying processes (*e.g*., conventional lyophilization, conventional spray drying, etc.) processes have rendered previous attempts to form dry bacteriophage products with acceptable levels of viable, or virulent bacteriophage particles unsuccessful.

In fact, initial attempts to convert liquid bacteriophage products to dry bacteriophage products were unsuccessful, as evidenced by the following example.

### EXAMPLE 1

Spray drying processes convert bulk liquid into a spray or fine mist, which eventually becomes a fine powder through the combined use of pressurized air and heat. The conversion of a bulk liquid into a spray or fine mist may be effected through a process called "atomization." Atomization can be performed through a variety of known techniques. Examples of such techniques include atomization through highpressure nozzles and rotary atomization.

Nozzle atomization systems generate the highest shearing forces, which start at a very high pressure feed pump, then channel the bulk liquid feed through a high precision orifice. Once the fine mist is safely injected through the orifice, the mist is heated. The mixing of the small droplets with the heat is a relatively slow process with a nozzle system, as is resulting transfer of heat to the droplet.

In rotary atomization systems, the pressures that are required and the shearing forces that are applied to a bulk liquid are greatly reduced from those used in nozzle atomization systems. Due to the complex atomization process, the number of moving parts increases slightly and repair costs are slightly higher. Overall, it was believed that rotary atomization would provide the best solution for drying bulk liquid bacteriophage product.

A rotary atomization study was performed using a PRODUCTION MINOR^{™} rotary atomization spray dryer, available from GEO Niro of Søborg, Denmark. Liquid bacteriophage products (*i.e*., fermentation products that included bacteriophage, fermentation media, and bacterial debris, or lysate) that included three different types of bacteriophage were used, each effective in controlling a different bacterial host: (1) bacteriophage effective against *Clavibacter michiganensis* pv. *michiganensis,* a plant pathogen (causes Canker on tomato plants); (2) bacteriophage effective against *Xanthomonas campestris* pv. *vesicatoria,* a plant pathogen (causes Spot on tomato and pepper plants); and (3) bacteriophage effective against *Escherichia coli* 0157:H7, a human pathogen (causes sickness and death among humans). In an effort to optimize the recovery of viable, or virulent, bacteriophage, the rotary atomization spray dryer was set to its lowest possible inlet and outlet temperature settings.

The process parameters and results are set forth below:

| **Run #** | | | **1** | | **2** | **3** | **4** | **5** | **6** | **7** | **8** | **9** | | **10** | **11** | |
|---|---|---|---|---|---|---|---|---|---|---|---|---|---|---|---|---|
| **NIRO TEST DATA** | | | | | | | | | | | | | | | | |
| **Date** | | | 5-Dec | | 5-Dec | 5-Dec | 5-Dec | 5-Dec | 5-Dec | 6-Dec | 6-Dec | 6-Dec | | 6-Dec | 6-Dec | |
| **Feed Material** | | | CMM | | | CMM+Starch | | | XCV+Milk | | | EC | | | EC+Starch | |
| | Feed Number | | 1 | | | 2 | | | 3 | | | 4 | | | | 5 |
| | % Solids (1) | | 133% | | | 296% | | | 251% | | | 196% | | | 452% | |
| **Feed Properties** | | | | | | | | | | | | | | | | |
| | % N-Lok 1930 Starch added | | - | | | 100 00% | | | - | | | - | | | 100 00% | |
| | 25% concentrated skim milk | | | | | | | | 2 5 Liter | | | | | | | |
| | Density (c/cm³) | | 1.010 | | | 1.010 | | | 1.010 | | | 1.040 | | | 1.010 | |
| | Density measured at 0°C | | 5.5 | | | 11.5 | | | 8.4 | | | 11.2 | | | 12. 9 | |
| | Viscosity (centipolse), AVG. (5) | | - | | - | - | - | - | - | - | - | - | | - | - | |
| | Sample/Spindle | | A | B | | A | B | | A | B | | A | B | | A | B |
| | | RPM 6 | 5.0 . 35.0 | | | 15.0 | 10.0 | | 5.0 | 0.0 | | 10.0 | 10.0 | | 0.0 | 0.0 |
| | | RPM.12 | 2.5 | 22.5 | | 12.5 | 17.5 | | 12.5 | 00 | | 15.0 | 10.0 | | 2.5 | 5.0 |
| | | RPM.30 | 7.5 | 8.0 | | 8.0 | 6.0 | | 8.0 | 3.0 | | 6.0 . 6.0 | | | 6.0 | 9.0 |
| | | RPM 60 | 4.5 | 4.0 | | 4.0 | 5.0 | | 5.5 | 3.5 | | 4.0 | 4.5 | | 55:5.5 | |
| | Spindle No | | 1 | | | 1 | | | 1 | | | 1 | | | 1 | |
| | Viscosity measured at 0' C | | 5.5 | | | 11.5 | | | 8.4 | | | 11.2 | | | 12.9 | |
| | pH | | 6.20 | | | 6.15 | | | 7.15 | | | 7.40 | | | 7.33 | |
| | pH measured 0°C | | 5.5 | | | 11.5 | | | 8.4 | | | 11.2 | | | 12.9 | |
| **Drying Conditions** | | | | | | | | | | | | | | | | |
| | Atomization Method | | Rotary, Vaned Wheel | | | | | | | | | | | | | |
| | Atomizer Speed (RPM) | | 30000 | | 30000 | 30000 | 20000 | 20000 | 20000 | 20000 | 20000 | 20000 | | 15000 | 15000 | |
| | Air Flow Rate (kg/hr) | | 438 | | 426 | 411 | 406 | 400 | 40C | 395 | 385 | 406 | | 406 | 411 | |
| | Inlet Temperature (°C) | | 199 | | 201 | 200 | 199 | 220 | 220 | 221 | 221 | 221 | | 222 | 222 | |
| | Outlet Temperature (°C) | | 91 | | 111 | 121 | 119 | 132 | 130 | 110 | 130 | 119 | | 119 | 110 | |
| | Amount Sprayed (kg) | | 19.38 | | 6 57 | 6.31 | 354 | 12 88 | 7.32 | 11.62 | 957 | 8.11 | | 9.15 | 9.09 | |
| | Spray Rate (kg/hr) | | 15.93 | | 1194 | 14.03 | 922 | 1226 | 9.76 | 1452 | 11.48 | 12.17 | | 18.30 | 1759 | |
| | Run Time (hr minutes) | | 1.13 | | 033 | 027 | 023 | 103 | 045 | 0.48 | 050 | 040 | | 0.30 | 0.31 | |
| **Product** | | | | | | | | | | | | | | | | |
| | Kilograms Produced | | "Granda" | | 0.115 | 0.080 | 0.072 | 0.278 | 0.143 | 0.173 | 0.168 | 0.155 | | 0.120 | 0271 | |
| | % Phage | | 100.00% | | 50.00% | 50 00% | 50.00% | 50.00% | 25.00% | 25.00% | 100.00% | 100 00% | | 100.00% | 50.00% | |
| | % Recovery | | - | | 59 18% | 42 82% | 68.62% | 73.01% | 77.91% | 59.20% | 89.58% | 97.49% | | 66.90% | 65.96% | |
| | Residual Moisture (%) (2) | | 9.17% | | 5071% | 5.41% | 5.54% | 4.37% | 5.50% | 8.85% | 5.17% | 5.20% | | 4.13% | 5.89% | |
| | Particle Size Distribution (3) | | | | | | | | | | | | | | | |
| | D 10 (10 wt % smaller than) | | - | | 1474 | 8.04 | 10.45 | 10.89 | 13.02 | 15.60 | 6.98 | 7.06 | | 8.26 | 10.38 | |
| | D 50 150 wt % smaller than) | | - | | 52.20 | 14.41 | 22.69 | 2052 | 3067 | 50.10 | 13.21 | 13.14 | | 1602 | 21.18 | |
| | D 90 (90 wt % smaller than) | | - | | 361.24 | 25.19 | 6761 | 48.98 | 138.91 | 123.16 | 2102 | 20.69 | | 25.56 | 3702 | |
| | Bulk Density (grams/cc) | | - | | 0.381 | 0.288 | 0.344 | 0.351 | 0.170 | 0.160 | 0282 | 0.270 | | 0.297 | 0 200 | |
| | Tapped Density (grams/cc) | | - | | 0.378 | 0.369 | 0.446 | 0.450 | 0 237 | 0.179 | 0.340 | 0 334 | | 0.366 | 0 263 | |
| **Notes*** | | | | | | | | | | | | | | | | |
| | (1) Sartorius Moisture Balance at 95 deg C for 10 minutes, 2 gram sample of the feed material | | | | | | | | | | | | | | | |
| | (2) Sartorius Moisture Balance at 95 deb C for 30 minutes, 1 gram sample of the product | | | | | | | | | | | | | | | |
| | (3) Hortbe LA=910 Laser Scattering Particle Size Analyzer | | | | | | | | | | | | | | | |
| | (41 The brush down material was a combination of the multiple runs (Run 6-7 and 8-11) | | | | | | | | | | | | | | | |
| | (5) Staren N-Lock 1930 added 1.1 ratio of original feed solids | | | | | | | | | | | | | | | |
| | (6) Brookfield LVG Viscometer | | | | | | | | | | | | | | | |

| **OL CALCULATED EXPECTATIONS** | | | | | | | | | | | | | | | | |
|---|---|---|---|---|---|---|---|---|---|---|---|---|---|---|---|---|
| Calculated Titers From Powder (Expected) | | | | | | | | | | | | | | | | |
| | Powder Sample "A" | | | | | | | | | | | | | | | |
| | | *100% Titer (Max + Binder)* | *N*/*A* | | *1.59E+10* | *220E+10* | *1.37E+10* | *1.29E+10* | *3.59E+10* | *4.71E+10* | *3.11E+09* | *2.86E+09* | | *4.16E+09* | *1.78E+09* | |
| | | *Titer after Phage Recovery %* | *N*/*A* | | *9.42E+09* | *9.41E+09* | *9.40E+09* | *9.43E+09* | *2.80E+10* | *2.79E+10* | *2.79E+09* | *2.79E+09* | | *2.79E+09* | *1.17E+09* | |

| **OL ACTUALS** | | | | | | | | | | | | | | | | |
|---|---|---|---|---|---|---|---|---|---|---|---|---|---|---|---|---|
| **Measured Titers From Powder** | | | | | | | | | | | | | | | | |
| | Powder Sample "A" (1/11/07) Test 1 | | | | 1.00E+05 | 3.60E+04 | 6.30E+04 | 1.20E+04 | 2.00E+05 | 1.00E+05 | 1.00E+05 | 1.20E+02 | | 1.20E+02 | 1.40E+02 | |
| | Powder Sample "B" (2/07/07) Test 1 | | | | 5.50E+04 | 3.10E+08 | 5.50E+04 | 1.30E+04 | 2.30E+05 | 1.10E+05 | 1.00E+05 | 1.10E+02 | | 1.63E+02 | 1.20E+02 | |

The predicted recovery rates of the dry bacteriophage product (a powder) for controlling the two plant pathogens were in the range of 1.29 × 10¹⁰ pfu/ml (low) to 4.71 × 10¹⁰ pfu/ml (high). Actual results were observed to be in the range of 1.20 × 10⁴ pfu/ml (low) to 3.1 × 10⁶ pfu/ml, or a 4 to 6 log reduction in viable, or virulent, bacteriophage. The bacteriophage effective against human pathogens faired even worse, with predicted recovery rates of the dry bacteriophage product in the range of 1.17 × 10⁹ pfu/ml (low) to 4.16 × 10⁹ pfu/ml (high) and actual results observed in the range of 1.10 × 10² pfu/ml (low) to 1.80 × 10² pfu/ml, representing a 7+ log reduction in viable, or virulent, bacteriophage. It should be noted that the most optimal process conditions were applied, and still the results were extremely disappointing.

Despite the high expectations for rotary spray drying processes and their subsequent and significant failure, another study was conducted to determine whether or not an acceptable dry bacteriophage product could be produced. In that study, pulse combustion drying processes, were used.

Pulse combustion drying may be effected in a manner that virtually eliminates any possible shear.

In some embodiments, the percent solids content of the liquid bacteriophage product may be increased before the liquid bacteriophage product is subjected to drying processes. For example, a carrier material may be pre-dissolved or otherwise mixed into the liquid bacteriophage product. Without limiting the scope of the present invention, the carrier material may include dried milk (*e.g*., nonfat powdered milk, etc.), trehalose, maltodextrin, or the like.

Known gas dynamic atomization techniques may be used with an extremely low feed pressure (*e.g.,* about 0.0690 bar); *i.e.,* the pressure at which a liquid bacteriophage product is introduced into a pulse combustion drying apparatus. In some embodiments, low feed pressure may be achieved by introducing the liquid bacteriophage product into the pulse combustion drying apparatus through an open pipe, rather than through a precision orifice or wheel, to a dryer.

In the drying process, droplets of the liquid bacteriophage product were nearly instantaneously exposed to heat.

By tailoring one or more of the type of carrier material, amount of carrier material, feed pump speed/pressure, combustion pressure (*e.g.*, about 0.10 bar (about 1.5 psi), etc.), contact temperature (*e.g*., about 540° C. (about 1020° F.) or less, etc.), exit temperature (*e.g*., about 82° C. (about 180° F.) or less, etc.), and the amount of time a liquid bacteriophage product and/or a dry bacteriophage product is exposed to an increased temperature, an environment can be created where bacteriophage recovery (*i.e*., viability, virulence, etc.) percentages are extremely high.

### EXAMPLE 2

In a study using pulse combustion drying, a fantastic discovery was made: bacteriophage recovery percentages were extremely high when compared with other techniques, including recovery percentages of about 1% or more, recovery percentages of about 10% or more, and, in some cases, up to 100% recovery was observed, thus highlighting the viability of pulse combustion drying as a technique for forming dry bacteriophage products.

Again, in this study, two of the three different types of liquid bacteriophage products (i.e., fermentation products that included bacteriophage, fermentation media, and bacterial debris, or lysate) used in the previous study were repeated here: (1) bacteriophage effective against *Xanthomonas campestris* pv. *vesicatoria,* a plant pathogen (causes Spot on tomato and pepper plants); and (2) bacteriophage effective against *Escherichia coli* 0157:H7, a human pathogen (causes sickness and death among humans). The solids contents of the liquid bacteriophage products were increased by the addition of carrier materials.

The tables that follow set forth the process parameters and results of various pulse combustion drying tests, which were effected by a pulse combustion dryer available from Pulse Combustion Systems of Payson, Arizona, in which liquid bacteriophage products were converted to dry bacteriophage products:

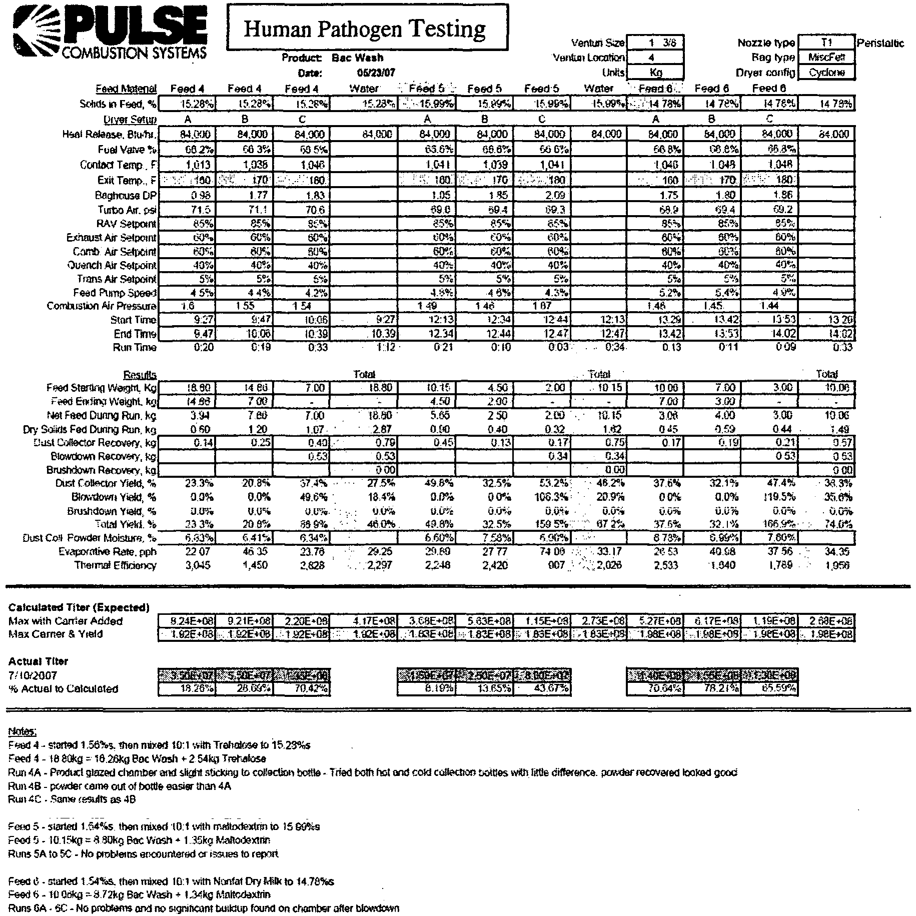

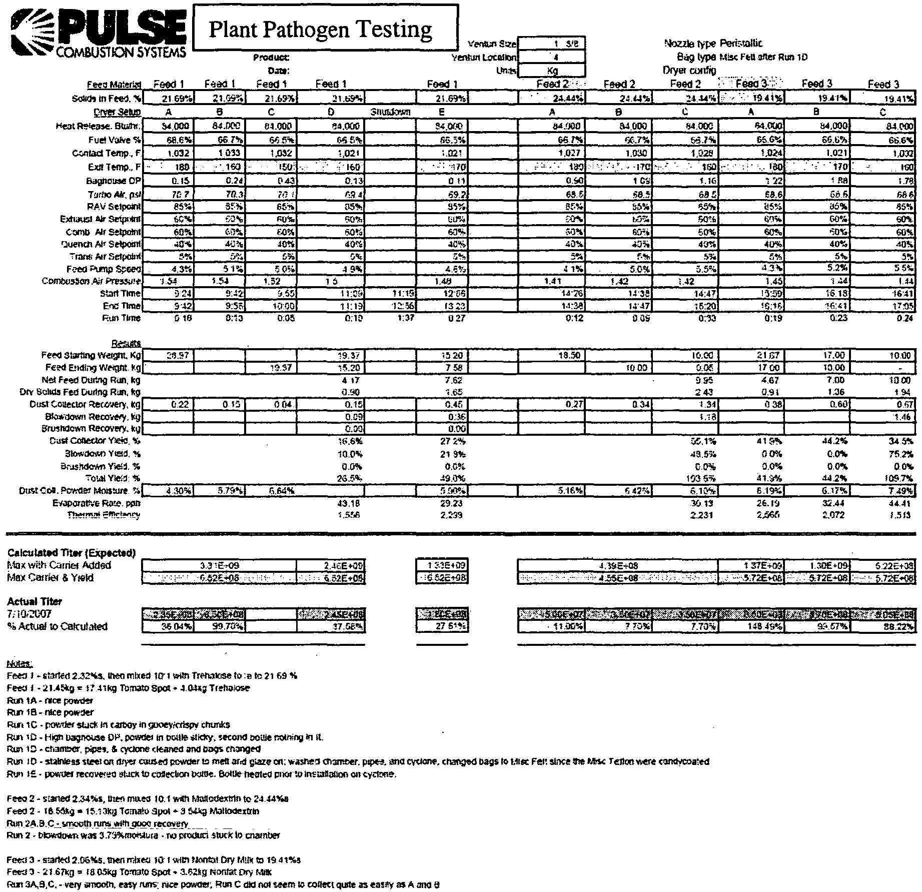

The predicted recovery rates of the dry powder (from the bacteriophage used to control the plant pathogen) were in the range of 4.55 × 10⁸ pfu/ml (low) to 6.52 × 10⁸ pfu/ml (high). Actual results were observed to be in the range of 3.50 × 10⁷ pfu/ml (low) to 8.50 × 10⁸ pfu/ml, or less than one log (10¹)reduction in viable, or virulent, bacteriophage in all cases, and within titering error (within 50%) in most cases. The bacteriophage effective against human pathogens produced similar results with predicted recovery rates of the dry powder in the range of 1.83 × 10⁸ pfu/ml (low) to 1.98 × 10⁸ pfu/ml (high) and actual results observed in the range of 1.50 × 10⁷ pfu/ml (low) to 1.40 × 10⁸ pfu/ml, again a one log (10¹) or less reduction in viable bacteriophage in all cases, and within titering error (within 50%) in many cases.

In various embodiments, the present invention includes the production of a highly effective dry bacteriophage product (*e.g*., in powder form) from a liquid bacteriophage product, which may comprise any combination of fermentation media, magnesium sulfate, lysate, bacteria, deionized, distilled or filtered tap water, lysozyme, DNAse, RNAse, and antifoaming agents, or through the manipulation of the described bacteriophage liquid through the addition of chemicals such as KOH or NaOH for the purposes of adjusting pH. In some embodiments, an optional carrier material (such as dried milk, trehalose, maltodextrin or the like) may be included in order to increase the percent solid content of the original feed material, while maintaining viability of the final powder. This can potentially increase the percentage of solids in the feed from a low percentage (*e.g*., 5% or less) to a much higher percentage (*e.g*., 20% or more). Embodiments in which no carrier material is used, or in which different types of carrier materials are used, are, of course, also within the scope of the present invention.

A wide variety of different types of bacteriophage may be dried in accordance with teachings of the present invention, including, without limitation, bacteriophage that are effective against human pathogens, animal pathogens, plant pathogens, or non-pathogenic strains of bacteria. This includes, but is not limited to, bacteriophage that are effective against one or more of the following plant pathogens: *Xanthomonas, Pseudomonas, Clavibacter, Ralstonia, Acidovorax, Erwinia, Burkholderia, Agrobacterium, Bacillus,* etc. It also includes, but is not limited to, bacteriophage that are effective against one or more of the following human pathogens: *Escherichia coli* 0157:H7, *Salmonella, Clostridium, Campylobacter, Listeria, Streptococcus, Staphylococcus, Helicobacter, Propionibacterium,* etc.

In some embodiments, spray drying processes including pulse combustion drying processes may be used. An effective pulse combustion drying process may, in some embodiments, employ minimal shearing forces, employ relatively low overall temperatures, and/or minimize the amount of time a liquid bacteriophage product is exposed to an increased temperature. These processes may be conducted in relatively short periods of time (*e.g*., less than ten hours, about two hours or less, about one hour or less) while substantially maintaining the viability, or virulence, of bacteriophage particles from the bulk liquid bacteriophage product (*e.g*., the dry bacteriophage product has at least one percent, at least ten percent, etc., of the viability, or virulence, of the bulk liquid bacteriophage product).

## Claims

1. A method for manufacturing a dry bacteriophage preparation, comprising subjecting a bulk liquid including a bacteriophage to pulse combustion drying.

2. The method of claim 1, wherein subjecting comprises subjecting a bulk liquid including the bacteriophage and at least one of a fermentation medium, a lysate, and a host bacteria to the pulse combustion drying.

3. The method of claim 1 or claim 2, further comprising:
introducing a carrier material into the bulk liquid to increase a solids content of the bulk liquid,
wherein subjecting comprises subjecting a bulk liquid including the carrier material to the pulse combustion drying.

4. The method of claim 3, wherein introducing the carrier material comprises adjusting a solids content of the bulk liquid to at least 20%.

5. The method of claim 3 or claim 4, wherein introducing the carrier material comprises introducing at least one of dried milk, trehalose, and maltodextrin into the bulk liquid.

6. The method of any of claims 1 through 5, wherein subjecting comprises subjecting the bulk liquid to a maximum pressure of 0.1 bar.

7. The method of any of claims 1 through 6, wherein subjecting comprises subjecting the bulk liquid to a contact temperature of 540° C or less.

8. The method of any of claims 1 through 7, wherein the pulse combustion drying process employs an exit temperature of 82° C. or less.

9. The method of any of claims 1 through 8, wherein subjecting the bulk liquid to pulse combustion drying results in, at most, a one log reduction in a number of viable bacteriophage particles from the bulk liquid to a dry bacteriophage product.

10. The method of any of claims 1 through 9, wherein the pulse combustion drying process is effected at a rate of one liter every ninety seconds or faster.

11. The method of any of claims 1 through 10, wherein the bulk liquid has a volume of up to twenty liters.

## Patentansprüche

1. Verfahren zur Herstellung eines trockenen Bakteriophagenpräparats, das umfasst, ein Flüssigkeitsvolumen, das einen Bakteriophage enthält, einer Impulsverbrennungstrockung auszusetzen.

2. Verfahren nach Anspruch 1, wobei das Aussetzen umfasst, ein Flüssigkeitsvolumen, das den Bakteriophage und ein Fermentierungsmedium und/oder ein Lysat und/oder ein Wirtsbakterium enthält, der Impulsverbrennungstrockung auszusetzen.

3. Verfahren nach Anspruch 1 oder Anspruch 2, das des Weiteren Folgendes umfasst:
Einleiten eines Trägermaterials in das Flüssigkeitsvolumen, um einen Feststoffgehalt des Flüssigkeitsvolumens zu erhöhen, wobei das Aussetzen umfasst, ein Flüssigkeitsvolumen, welches das Trägermaterial enthält, der Impulsverbrennungstrockung auszusetzen.

4. Verfahren nach Anspruch 3, wobei das Einleiten des Trägermaterials umfasst, einen Feststoffgehalt des Flüssigkeitsvolumens auf mindestens 20 % einzustellen.

5. Verfahren nach Anspruch 3 oder Anspruch 4, wobei das Einleiten des Trägermaterials umfasst, Trockenmilch und/oder Trehalose und/oder Maltodextrin in das Flüssigkeitsvolumen einzuleiten.

6. Verfahren nach einem der Ansprüche 1 bis 5, wobei das Aussetzen umfasst, das Flüssigkeitsvolumen einem maximalen Druck von 0,1 bar auszusetzen.

7. Verfahren nach einem der Ansprüche 1 bis 6, wobei das Aussetzen umfasst, das Flüssigkeitsvolumen einer Kontakttemperatur von maximal 540 °C auszusetzen.

8. Verfahren nach einem der Ansprüche 1 bis 7, wobei für den Impulsverbrennungstrockungsprozess eine Austrittstemperatur von maximal 82 °C verwendet wird.

9. Verfahren nach einem der Ansprüche 1 bis 8, wobei die Impulsverbrennungstrockung des Flüssigkeitsvolumens maximal zu einer einzelnen Logstufenreduzierung einer Anzahl lebensfähiger Bakteriophagenteilchen von dem Flüssigkeitsvolumen zu einem trockenen Bakteriophagenprodukt führt.

10. Verfahren nach einem der Ansprüche 1 bis 9, wobei der Impulsverbrennungstrockungsprozess mit einer Rate von einem Liter alle neunzig Sekunden oder schneller ausgeführt wird.

11. Verfahren nach einem der Ansprüche 1 bis 10, wobei das Flüssigkeitsvolumen ein Volumen von bis zu zwanzig Litern hat.

## Revendications

1. Procédé de fabrication d'une préparation de bactériophage anhydre, comprenant la soumission d'un liquide en masse comprenant un bactériophage à un séchage par combustion pulsée.

2. Procédé selon la revendication 1, dans lequel la soumission comprend la soumission d'un liquide en masse comprenant le bactériophage et au moins un d'un milieu de fermentation, un lysat et une bactérie hôte au séchage par combustion pulsée.

3. Procédé selon la revendication 1 ou 2, comprenant en outre :
l'introduction d'une matière véhicule dans le liquide en masse pour augmenter une teneur en solides du liquide en masse, dans lequel la soumission comprend la soumission d'un liquide en masse comprenant la matière véhicule au séchage par combustion pulsée.

4. Procédé selon la revendication 3, dans lequel l'introduction de la matière véhicule comprend d'ajuster une teneur en solides du liquide en masse à au moins 20 %.

5. Procédé selon la revendication 3 ou la revendication 4, dans lequel l'introduction de la matière véhicule comprend l'introduction d'au moins un parmi le lait anhydre, le tréhalose et la maltodextrine dans le liquide en masse.

6. Procédé selon l'une quelconque des revendications 1 à 5, dans lequel la soumission comprend la soumission du liquide en masse à une pression maximum de 0,1 bar.

7. Procédé selon l'une quelconque des revendications 1 à 6, dans lequel la soumission comprend la soumission du liquide en masse à une température de contact de 540 °C ou moins.

8. Procédé selon l'une quelconque des revendications 1 à 7, dans lequel le processus de séchage par combustion pulsée emploie une température de sortie de 82 °C ou moins.

9. Procédé selon l'une quelconque des revendications 1 à 8, dans lequel la soumission du liquide en masse à un séchage par combustion pulsée conduit à, au plus, une réduction d'un log d'un nombre de particules viables du liquide en masse à un produit de bactériophage anhydre.

10. Procédé selon l'une quelconque des revendications 1 à 9, dans lequel le processus de séchage par combustion pulsée est effectué à une vitesse de un litre chaque quatre vingt dix secondes ou plus rapidement.

11. Procédé selon l'une quelconque des revendications 1 à 9, dans lequel le liquide en masse a un volume de jusqu'à vingt litres.
